# EUROPEAN PATENT APPLICATION

(11) **EP 4 781 990 A1**
(43) Date of publication of application: **29.07.2026**
(21) Application number: 24867421.0
(22) Date of filing: 18.09.2024
(51) Int. Cl.: A61K 31/496, A61K 31/444, A61K 31/4412, A61K 9/48, A61K 9/20, A61K 9/28, A61P 35/00

(54) **PHARMACEUTICAL COMPOSITION CONTAINING HETEROARYL OXYNAPHTHALENE COMPOUND, PREPARATION METHOD THEREFOR, AND USE THEREOF**

(30) Priority: 18.09.2023 CN 202311201152
(71) Applicant: Shanghai Runshi Medical Technology Co., Ltd, Shanghai 201318 (CN); Shanghai Institute of Materia Medica, Chinese Academy of Sciences, Shanghai 201203 (CN)
(72) Inventor: BAI, Jing, Shanghai 201318 (CN); ZHANG, Ao, Shanghai 201203 (CN); ZHENG, Yang, Shanghai 201318 (CN); GENG, Meiyu, Shanghai 201203 (CN); YIN, Lu, Shanghai 201318 (CN); SONG, Zilan, Shanghai 201203 (CN); LI, Xiue, Shanghai 201318 (CN); AI, Jing, Shanghai 201203 (CN); XUN, Haohua, Shanghai 201318 (CN); WEN, Shilong, Shanghai 201318 (CN)
(74) Representative: Gille Hrabal Partnerschaftsgesellschaft mbB Patentanwälte
(86) International application number: PCT/CN2024/119329
(87) International publication number: WO 2025/061013

(57) **Abstract**

The invention relates to a pharmaceutical composition containing a compound (A), a preparation method therefor, and a use thereof. The preparation method for the pharmaceutical composition is simple and suitable for industrial large-scale production, and tablets prepared from the pharmaceutical composition have good active ingredient content uniformity and a good active ingredient dissolution, have stable properties, and are suitable for long-term storage.

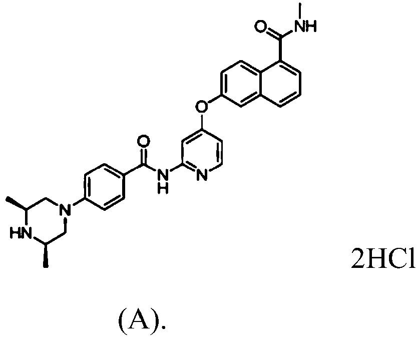

## Description

### TECHNICAL FIELD

The present disclosure pertains to the field of pharmaceutics, and particularly relates to a pharmaceutical composition comprising a heteroaryloxynaphthalene compound, a preparation method therefor, and use thereof.

### BACKGROUND

Fibroblast growth factor receptors (FGFRs) are important molecular typing targets of tumors, and are abnormally activated in various tumors, especially refractory tumors and Chinese characteristic tumor species. Currently, most of clinical studies on inhibitors targeting FGFR are in an early stage, and the FGFR inhibitors in clinical research are mainly multi-target, have weak inhibitory activity against FGFR, and often enhance antagonistic effects on kinase insert domain containing receptors (KDRs), receptors of human vascular endothelial growth factors, causing serious adverse effects and greatly limiting the application of the anti-tumor effects of the inhibitors targeting FGFR and the maximization of the clinical efficacy. However, FGFR and KDR have the ability to compensate for activation, suggesting that they compensate for each other to mediate drug resistance. In fact, studies have reported that the activation of FGFR is a drug resistance mechanism of KDR inhibitors. In addition, the role of the KDR family in strengthening immunosuppression and promoting tumors has also been gradually revealed in recent years. Therefore, the development of inhibitors co-targeting FGFR and KDR has important clinical application potential.

It should not be ignored that in recent years, tumor therapy is not merely limited to tumor cells themselves, and the role of the inseparable tumor microenvironment, as a functional whole, in promoting tumor progression and mediating drug resistance attracts great attention. Among them, tumor-associated macrophages (TAMs) are important microenvironment stromal cells, which not only directly inhibit effector T cells from exerting killing effects and synergistically promote tumor immunosuppressive agent microenvironment, but also promote the growth and metastasis of tumor cells and mediate drug resistance in tumors by promoting angiogenesis in tumors through multiple processes. Colony stimulating factor-1 receptor (CSF-1R) is expressed in macrophages and is crucial for maintenance of TAM differentiation towards M2 polarized phenotype, and its proliferation and survival. Based on this, synchronously targeting FGFR/KDR/CSF-1R can not only antagonize tumor cells themselves, but also regulate the tumor microenvironment. This facilitates antagonism of tumors through multiple processes, remodels the immunosuppressive microenvironment, and delays the onset of acquired drug resistance.

A compound of formula (I) is a novel FGFR/CSF1R/KDR-targeting small molecule inhibitor developed based on the above mechanism, and pre-clinical pharmacological studies show that the compound has relatively good efficacy in various tumor models.

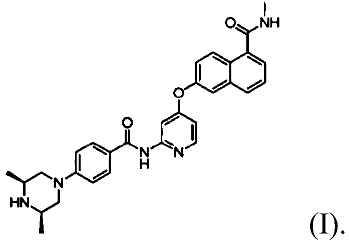

The PCT patent document WO2017/140269A1 discloses a structure of the compound of formula (I), a preparation method therefor, and pharmaceutical use thereof in preventing and/or treating a disease associated with FGFR.

The PCT patent document WO2021/170078A1 discloses use of the compound of formula (I) as a CSF-1R inhibitor.

None of the patent documents described above have investigated the formulation formulas of the compound of formula (I) and the pharmaceutically acceptable salt thereof, and preparation thereof. In view of the great application prospect of the compound of formula (I) as an FGFR/CSF1R/KDR-targeting inhibitor in the treatment of tumors, there is a need to provide a pharmaceutical composition comprising the compound of formula (I) or the pharmaceutically acceptable salt thereof with good druggability.

### SUMMARY

One object of the present disclosure is to provide a pharmaceutical composition comprising a compound of formula (I) or a pharmaceutically acceptable salt thereof as an active ingredient with a simple preparation method and suitable for industrial production.

Another object of the present disclosure is to provide a pharmaceutical composition comprising a compound of formula (I) or a pharmaceutically acceptable salt thereof as an active ingredient with good druggability. Another object of the present disclosure is to provide a pharmaceutical composition comprising a compound of formula (I) or a pharmaceutically acceptable salt thereof as an active ingredient, which can be used for preparing a solid oral formulation with a good active ingredient dissolution and good active ingredient content uniformity.

Another object of the present disclosure is to provide a pharmaceutical composition comprising a compound of formula (I) or a pharmaceutically acceptable salt thereof as an active ingredient, which has excellent stability and is suitable for long-term storage.

In order to achieve the objects of the disclosure described above, the inventors of the present application have found through previous research that a dihydrochloride salt of a compound of formula (I), compound (A), is easy to dry and has good solubility, stable quality, thermodynamic stability, and ease of druggability, and is suitable for use as an active ingredient of a pharmaceutical composition. Further, the inventors also performed screening experiments on other components in the pharmaceutical composition and found a formula of a specific pharmaceutical composition that can meet one or more of the requirements described above, thereby completing the present disclosure.

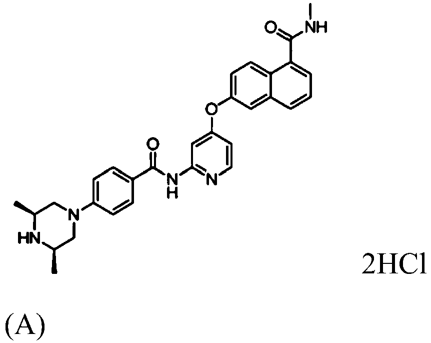

A first aspect of the present disclosure provides a pharmaceutical composition comprising the following components in percentage by weight of the pharmaceutical composition:
0.1%-10% of a compound (A) having a structure of formula (A) below: 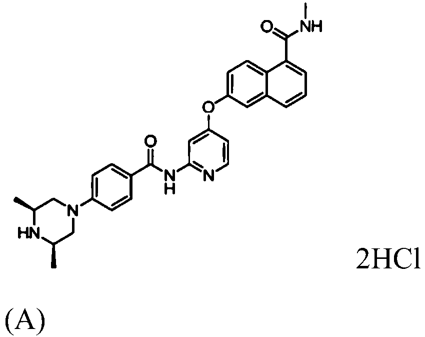
70%-95% of a filler;
0-15% of a disintegrant;
0-3% of a glidant; and
0.5-5% of a lubricant.

The pharmaceutical composition of the present disclosure comprises the compound (A) as an active ingredient.

In some embodiments of the present disclosure, the compound (A) is in a crystalline form.

In some embodiments of the present disclosure, the crystalline form of the compound (A) is a crystalline form I, wherein the crystalline form I has characteristic peaks at 2θ of 5.0±0.2°, 13.5±0.2°, 19.3±0.2°, 20.5±0.2°, 21.4±0.2°, and 25.2±0.2° in an X-ray powder diffraction pattern obtained using Cu-Kα radiation, or
the crystalline form I has characteristic peaks at 2θ of 5.0±0.2°, 10.4±0.2°, 13.5±0.2°, 19.3±0.2°, 20.5±0.2°, 21.4±0.2°, 23.4±0.2°, and 25.2±0.2° in an X-ray powder diffraction pattern obtained using Cu-Kα radiation, or
the crystalline form I has characteristic peaks at 2θ of 5.0±0.2°, 10.4±0.2°, 13.5±0.2°, 19.3±0.2°, 20.5±0.2°, 21.4±0.2°, 22.5±0.2°, 23.4±0.2°, 23.9±0.2°, 24.3±0.2°, and 25.2±0.2° in an X-ray powder diffraction pattern obtained using Cu-Kα radiation, or
the crystalline form I has characteristic peaks at 2θ of 5.0±0.2°, 10.4±0.2°, 11.2±0.2°, 13.5±0.2°, 19.3±0.2°, 20.5±0.2°, 21.4±0.2°, 22.0±0.2°, 22.5±0.2°, 23.4±0.2°, 23.9±0.2°, 24.3±0.2°, 25.2±0.2°, and 26.6±0.2° in an X-ray powder diffraction pattern obtained using Cu-Kα radiation, or
the crystalline form I has characteristic peaks at 2θ of 5.0±0.2°, 10.0±0.2°, 10.4±0.2°, 11.2±0.2°, 13.5±0.2°, 14.1±0.2°, 15.7±0.2°, 17.4±0.2°, 18.0±0.2°, 19.3±0.2°, 20.1±0.2°, 20.5±0.2°, 21.4±0.2°, 22.0±0.2°, 22.5±0.2°, 23.4±0.2°, 23.9±0.2°, 24.3±0.2°, 25.2±0.2°, 26.6±0.2°, 28.6±0.2°, and 30.1±0.2° in an X-ray powder diffraction pattern obtained using Cu-Kα radiation, or
the crystalline form I has characteristic peaks at 2θ of 5.0±0.2°, 10.0±0.2°, 10.4±0.2°, 11.2±0.2°, 13.5±0.2°, 14.1±0.2°, 15.7±0.2°, 17.4±0.2°, 18.0±0.2°, 19.3±0.2°, 20.1±0.2°, 20.5±0.2°, 21.4±0.2°, 22.0±0.2°, 22.5±0.2°, 23.4±0.2°, 23.9±0.2°, 24.3±0.2°, 25.2±0.2°, 26.6±0.2°, 28.6±0.2°, 30.1±0.2°, 32.0±0.2°, and 33.0±0.2° in an X-ray powder diffraction pattern obtained using Cu-Kα radiation, or
The crystalline form I has X-ray powder diffraction analysis data substantially as shown in Table 2 or 3 below.

The preparation, testing, and other related details of the compound (A) and the crystalline form I thereof can be found in Patent Application No. CN202310263447.7 or PCT/CN2023/082140, the entire contents of which are incorporated herein by reference.

In some embodiments of the present disclosure, the compound (A) accounts for 1%-10%, preferably 2%-8%, more preferably 2%-7%, further preferably 2%-6%, even further preferably 2%-5%, and even further preferably 2%-4% in percentage by weight of the pharmaceutical composition.

The pharmaceutical composition of the present disclosure comprises one or more fillers. "Filler" described herein refers to an inert substance used to provide the desired volume, flowability, and compression properties in the preparation of a solid dosage form. In an embodiment of the present disclosure, the filler is selected from one or more of microcrystalline cellulose, silicified microcrystalline cellulose, xylitol, mannitol, sorbitol, starch, pregelatinized starch, dextrin, lactose, sucrose, glucose, fructose, maltose, calcium carbonate, calcium sulfate, and dicalcium phosphate; preferably, the filler is one or more of microcrystalline cellulose, silicified microcrystalline cellulose, mannitol, starch, pregelatinized starch, or dextrin; more preferably, the filler is one or more of microcrystalline cellulose, silicified microcrystalline cellulose, mannitol, lactose, or pregelatinized starch; further preferably, the filler is one of microcrystalline cellulose or silicified microcrystalline cellulose, a combination of one of microcrystalline cellulose or silicified microcrystalline cellulose and one of mannitol or lactose (e.g., a combination of microcrystalline cellulose and mannitol, a combination of microcrystalline cellulose and lactose, a combination of silicified microcrystalline cellulose and mannitol, or a combination of silicified microcrystalline cellulose and lactose), or a combination of one of microcrystalline cellulose or silicified microcrystalline cellulose, one of mannitol or lactose, and pregelatinized starch (e.g., a combination of microcrystalline cellulose, mannitol, and pregelatinized starch; a combination of microcrystalline cellulose, lactose, and pregelatinized starch; a combination of silicified microcrystalline cellulose, mannitol, and pregelatinized starch; a combination of silicified microcrystalline cellulose, lactose, and pregelatinized starch); even further preferably, the filler is a combination of microcrystalline cellulose, mannitol, and pregelatinized starch. In some embodiments of the present disclosure, the filler accounts for 75%-95%, preferably 80%-95%, more preferably 85%-95%, and further preferably 85%-92% in percentage by weight of the pharmaceutical composition.

In some embodiments of the present disclosure, when the filler is selected from a combination of one of microcrystalline cellulose or silicified microcrystalline cellulose and one of mannitol or lactose, the mannitol or lactose has a weight percentage of 15-50%, preferably 20-45%, more preferably 20%-40%, and further preferably 20%-30%; the microcrystalline cellulose or silicified microcrystalline cellulose has a weight percentage of 40-75%, preferably 50-75%, more preferably 60-75%, and further preferably 65-75%; the mannitol or lactose and the microcrystalline cellulose or silicified microcrystalline cellulose are in a weight ratio of 1:5 to 1:1, preferably 1:4 to 1:1, more preferably 1:4 to 1:1.5, further preferably 1:3.6 to 1:1.5, even further preferably 1:3.6 to 1:2.8, and even further preferably 1:3.6 to 1:3.

In some embodiments of the present disclosure, when the filler is selected from a combination of one of microcrystalline cellulose or silicified microcrystalline cellulose, one of mannitol or lactose, and pregelatinized starch, the mannitol or lactose has a weight percentage of 15-45%, preferably 20-45%, more preferably 20%-41%, and further preferably 20%-30%; the microcrystalline cellulose or silicified microcrystalline cellulose has a weight percentage of 35-70%, preferably 40%-65%; the mannitol or lactose and the microcrystalline cellulose or silicified microcrystalline cellulose are in a weight ratio of 1:5 to 1:1, preferably 1:4 to 1:1, more preferably 1:4 to 1:1.5, further preferably 1:3.6 to 1:1.5, even further preferably 1:3.6 to 1:2.8, even further preferably 1:3.3 to 1:2.8, and even further preferably 1:3.05; the pregelatinized starch has a weight percentage of 5-25%, preferably 5-15%, more preferably 8-12%, further preferably 10-11%, and even further preferably 10.5%.

The pharmaceutical composition of the present disclosure may comprise one or more disintegrants. "Disintegrant" described herein refers to a substance used in a solid dosage form to facilitate the breaking of the solid into smaller particles that are more easily dispersed or dissolved. In an embodiment of the present disclosure, the disintegrant is selected from: sodium carboxymethyl starch, sodium carboxymethylcellulose, croscarmellose sodium, dry starch, low-substituted hydroxypropylcellulose, polyvinylpyrrolidone, crospovidone, effervescent disintegrant, polacrilin potassium, and sodium alginate; preferably, the disintegrant is sodium carboxymethyl starch, sodium carboxymethylcellulose, croscarmellose sodium, low-substituted hydroxypropylcellulose, polyvinylpyrrolidone, or crospovidone; more preferably, the disintegrant is sodium carboxymethyl starch, low-substituted hydroxypropylcellulose, or crospovidone; further preferably, the disintegrant is crospovidone.

In some embodiments of the present disclosure, the disintegrant accounts for 0-10%, preferably 1%-6%, more preferably 2%-6%, more preferably 2%-5%, more preferably 2%-4%, further preferably 3%-5%, even further preferably 3.5%-4.5%, and even further preferably 3%-4% in percentage by weight of the pharmaceutical composition.

The pharmaceutical composition of the present disclosure comprises one or more glidants. "Glidant" described herein refers to a reagent used in a solid dosage form to facilitate the flowability of the solid. In an embodiment of the present disclosure, the glidant is selected from: talc, silica, and colloidal silica; preferably, the glidant is silica or colloidal silica; more preferably, the glidant is colloidal silica.

In some embodiments of the present disclosure, the glidant accounts for 0.5-3.0%, preferably 0.5%-2.5%, more preferably 0.5%-2%, and further preferably 1-2% in percentage by weight of the pharmaceutical composition.

The pharmaceutical composition of the present disclosure comprises one or more lubricants. "Lubricant" described herein refers to a substance used to reduce friction between particles and between a particle and a die orifice of a pharmaceutical composition. In an embodiment of the present disclosure, the lubricant is selected from: calcium stearate, magnesium stearate, zinc stearate, talc, sodium stearyl fumarate, stearic acid, glyceryl behenate, palmitic acid, glyceryl palmitostearate, magnesium lauryl sulfate, hydrogenated vegetable oil, sodium dodecyl sulfate, magnesium dodecyl sulfate, polyethylene glycol, colloidal silicon dioxide, and aluminum hydroxide; preferably, the lubricant is glyceryl behenate, magnesium stearate, or sodium stearyl fumarate; more preferably, the lubricant is glyceryl behenate or sodium stearyl fumarate.

In some embodiments of the present disclosure, the lubricant accounts for 0.5%-4%, preferably 1%-4%, more preferably 1%-3%, and further preferably 0.8%-3% in percentage by weight of the pharmaceutical composition.

In some embodiments of the present disclosure, the compound (A) accounts for 2%-5% in percentage by weight of the pharmaceutical composition, the filler accounts for 85%-95% in percentage by weight of the pharmaceutical composition, the disintegrant accounts for 2-6% in percentage by weight of the pharmaceutical composition, the glidant accounts for 0.5-3.0% in percentage by weight of the pharmaceutical composition, and the lubricant accounts for 0.8%-3% in percentage by weight of the pharmaceutical composition.

In some embodiments of the present disclosure, the compound (A) accounts for 2%-4% in percentage by weight of the pharmaceutical composition, the filler accounts for 85%-92% in percentage by weight of the pharmaceutical composition, the disintegrant accounts for 3-4% in percentage by weight of the pharmaceutical composition, the glidant accounts for 1-2% in percentage by weight of the pharmaceutical composition, and the lubricant accounts for 1%-3% in percentage by weight of the pharmaceutical composition.

In some embodiments of the present disclosure, the compound (A) accounts for 2%-5% in percentage by weight of the pharmaceutical composition, the filler accounts for 85%-95% in percentage by weight of the pharmaceutical composition, the disintegrant accounts for 2-6% in percentage by weight of the pharmaceutical composition, the glidant accounts for 0-3.0% in percentage by weight of the pharmaceutical composition, and the lubricant accounts for 0.8%-3% in percentage by weight of the pharmaceutical composition,
wherein the filler is selected from one of microcrystalline cellulose or silicified microcrystalline cellulose, a combination of one of microcrystalline cellulose or silicified microcrystalline cellulose and one of mannitol or lactose (e.g., a combination of microcrystalline cellulose and mannitol, a combination of microcrystalline cellulose and lactose, a combination of silicified microcrystalline cellulose and mannitol, and a combination of silicified microcrystalline cellulose and lactose), and a combination of one of microcrystalline cellulose or silicified microcrystalline cellulose, one of mannitol or lactose, and pregelatinized starch (e.g., a combination of microcrystalline cellulose, mannitol, and pregelatinized starch; a combination of microcrystalline cellulose, lactose, and pregelatinized starch; a combination of silicified microcrystalline cellulose, mannitol, and pregelatinized starch; a combination of silicified microcrystalline cellulose, lactose, and pregelatinized starch); the disintegrant is selected from sodium carboxymethyl starch, sodium carboxymethylcellulose, croscarmellose sodium, dry starch, low-substituted hydroxypropylcellulose, polyvinylpyrrolidone, crospovidone, effervescent disintegrant, polacrilin potassium, and sodium alginate; the glidant is selected from talc, silica, and colloidal silica; the lubricant is selected from calcium stearate, magnesium stearate, zinc stearate, talc, sodium stearyl fumarate, stearic acid, glyceryl behenate, palmitic acid, glyceryl palmitostearate, magnesium lauryl sulfate, hydrogenated vegetable oil, sodium dodecyl sulfate, magnesium dodecyl sulfate, polyethylene glycol, colloidal silicon dioxide, and aluminum hydroxide;
when the filler is selected from a combination of one of microcrystalline cellulose or silicified microcrystalline cellulose and one of mannitol or lactose, the mannitol or lactose has a weight percentage of 15-50%, preferably 20-45%, more preferably 20%-40%, and further preferably 20%-30%; the microcrystalline cellulose or silicified microcrystalline cellulose has a weight percentage of 40-75%, preferably 50-75%, more preferably 60-75%, and further preferably 65-75%; the mannitol or lactose and the microcrystalline cellulose or silicified microcrystalline cellulose are in a weight ratio of 1:5 to 1:1, preferably 1:4 to 1:1, more preferably 1:4 to 1:1.5, further preferably 1:3.6 to 1:1.5, even further preferably 1:3.6 to 1:2.8, and even further preferably 1:3.6 to 1:3; or
when the filler is selected from a combination of one of microcrystalline cellulose or silicified microcrystalline cellulose, one of mannitol or lactose, and pregelatinized starch, the mannitol or lactose has a weight percentage of 15-45%, preferably 20-45%, more preferably 20%-41%, and further preferably 20%-30%; the microcrystalline cellulose or silicified microcrystalline cellulose has a weight percentage of 35-70%, preferably 40%-65%; the mannitol or lactose and the microcrystalline cellulose or silicified microcrystalline cellulose are in a weight ratio of 1:5 to 1:1, preferably 1:4 to 1:1, more preferably 1:4 to 1:1.5, further preferably 1:3.6 to 1:1.5, even further preferably 1:3.6 to 1:2.8, even further preferably 1:3.3 to 1:2.8, and even further preferably 1:3.05; the pregelatinized starch has a weight percentage of 5-25%, preferably 5-15%, more preferably 8-12%, further preferably 10-11%, and even further preferably 10.5%.

In some embodiments of the present disclosure, the compound (A) accounts for 2%-4% in percentage by weight of the pharmaceutical composition, the filler accounts for 85%-92% in percentage by weight of the pharmaceutical composition, the disintegrant accounts for 2-6% in percentage by weight of the pharmaceutical composition, the glidant accounts for 0.5-3.0% in percentage by weight of the pharmaceutical composition, and the lubricant accounts for 0.8%-3% in percentage by weight of the pharmaceutical composition,
wherein the filler is selected from one of microcrystalline cellulose or silicified microcrystalline cellulose, a combination of one of microcrystalline cellulose or silicified microcrystalline cellulose and one of mannitol or lactose (e.g., a combination of microcrystalline cellulose and mannitol, a combination of microcrystalline cellulose and lactose, a combination of silicified microcrystalline cellulose and mannitol, and a combination of silicified microcrystalline cellulose and lactose), and a combination of one of microcrystalline cellulose or silicified microcrystalline cellulose, one of mannitol or lactose, and pregelatinized starch (e.g., a combination of microcrystalline cellulose, mannitol, and pregelatinized starch; a combination of microcrystalline cellulose, lactose, and pregelatinized starch; a combination of silicified microcrystalline cellulose, mannitol, and pregelatinized starch; a combination of silicified microcrystalline cellulose, lactose, and pregelatinized starch); the disintegrant is selected from sodium carboxymethyl starch, sodium carboxymethylcellulose, croscarmellose sodium, low-substituted hydroxypropylcellulose, polyvinylpyrrolidone, and crospovidone; the glidant is selected from talc, silica, and colloidal silica; the lubricant is selected from magnesium stearate, sodium stearyl fumarate, and glyceryl behenate;
when the filler is selected from a combination of one of microcrystalline cellulose or silicified microcrystalline cellulose and one of mannitol or lactose, the mannitol or lactose has a weight percentage of 15-50%, preferably 20-45%, more preferably 20%-40%, and further preferably 20%-30%; the microcrystalline cellulose or silicified microcrystalline cellulose has a weight percentage of 40-75%, preferably 50-75%, more preferably 60-75%, and further preferably 65-75%; the mannitol or lactose and the microcrystalline cellulose or silicified microcrystalline cellulose are in a weight ratio of 1:5 to 1:1, preferably 1:4 to 1:1, more preferably 1:4 to 1:1.5, further preferably 1:3.6 to 1:1.5, even further preferably 1:3.6 to 1:2.8, and even further preferably 1:3.6 to 1:3; or
when the filler is selected from a combination of one of microcrystalline cellulose or silicified microcrystalline cellulose, one of mannitol or lactose, and pregelatinized starch, the mannitol or lactose has a weight percentage of 15-45%, preferably 20-45%, more preferably 20%-41%, and further preferably 20%-30%; the microcrystalline cellulose or silicified microcrystalline cellulose has a weight percentage of 35-70%, preferably 40%-65%; the mannitol or lactose and the microcrystalline cellulose or silicified microcrystalline cellulose are in a weight ratio of 1:5 to 1:1, preferably 1:4 to 1:1, more preferably 1:4 to 1:1.5, further preferably 1:3.6 to 1:1.5, even further preferably 1:3.6 to 1:2.8, even further preferably 1:3.3 to 1:2.8, and even further preferably 1:3.05; the pregelatinized starch has a weight percentage of 5-25%, preferably 5-15%, more preferably 8-12%, further preferably 10-11%, and even further preferably 10.5%.

In some embodiments of the present disclosure, the compound (A) accounts for 2%-4% in percentage by weight of the pharmaceutical composition, the filler accounts for 85%-92% in percentage by weight of the pharmaceutical composition, the disintegrant accounts for 3-4% in percentage by weight of the pharmaceutical composition, the glidant accounts for 1-2% in percentage by weight of the pharmaceutical composition, and the lubricant accounts for 1%-3% in percentage by weight of the pharmaceutical composition, wherein the filler is selected from a combination of microcrystalline cellulose, mannitol, and pregelatinized starch, the mannitol has a weight percentage of 20-45%, the microcrystalline cellulose has a weight percentage of 40%-65%, the mannitol and the microcrystalline cellulose are in a weight ratio of 1:3.5 to 1:1, and the pregelatinized starch has a weight percentage of 5-25%; the disintegrant is selected from crospovidone; the glidant is selected from colloidal silica; the lubricant is selected from glyceryl behenate and sodium stearyl fumarate.

In some embodiments of the present disclosure, the compound (A) accounts for 2%-4% in percentage by weight of the pharmaceutical composition, the filler accounts for 85%-92% in percentage by weight of the pharmaceutical composition, the disintegrant accounts for 3-4% in percentage by weight of the pharmaceutical composition, the glidant accounts for 1-2% in percentage by weight of the pharmaceutical composition, and the lubricant accounts for 1%-3% in percentage by weight of the pharmaceutical composition, wherein the filler is selected from a combination of microcrystalline cellulose, mannitol, and pregelatinized starch, the mannitol has a weight percentage of 20-45%, the microcrystalline cellulose has a weight percentage of 40%-65%, the mannitol and the microcrystalline cellulose are in a weight ratio of 1:3.3 to 1:2.8, and the pregelatinized starch has a weight percentage of 5-15%; the disintegrant is selected from crospovidone; the glidant is selected from colloidal silica; the lubricant is selected from glyceryl behenate and sodium stearyl fumarate.

In some embodiments of the present disclosure, the pharmaceutical composition may further comprise an acidic pH regulator to regulate the pH of a microenvironment of the composition and increase stability of the composition. The acidic pH regulator is selected from oxalic acid, maleic acid, fumaric acid, glutaric acid, and the like, and accounts for 0-3% in percentage by weight of the pharmaceutical composition.

The sum of the weight percentages of all the components in the pharmaceutical composition of the present disclosure is 100%.

A second aspect of the present disclosure provides a solid oral formulation prepared from the pharmaceutical composition described above. "Solid oral formulation" described herein refers to a solid pharmaceutical formulation for oral administration, which is preferably a capsule or a tablet, more preferably a tablet, and further preferably a film-coated tablet.

In some embodiments of the present disclosure, the solid oral formulation is a film-coated tablet. A coating material used for the film-coated tablet is a gastrosoluble coating material, preferably comprises one or more of hydroxypropyl methylcellulose, polyvinyl alcohol, or polyvinyl alcohol polyethylene glycol copolymer, and may further comprise one or more of an opacifier (e.g., titanium dioxide), a plasticizer (e.g., polyethylene glycol, glycerol, propylene glycol, diethyl phthalate, diethyl ortho-phthalate, glycerol triacetate, triethyl citrate, glyceryl mono and dicaprylocaprate, etc.), a lubricant (e.g., talc), or a colorant (e.g., titanium aluminium lake, allura red aluminum lake, or brilliant blue aluminum lake). The coating material can also be commercially available, and a premix of a suitable coating material can be selected from Opadry 03B28796 and Opadry 321A610052.

In some embodiments of the present disclosure, a film coating weight gain is 1%-5%, preferably 2%-4%, e.g., 2%, 2.6%, 3%, 3.7%, or 4%, of a tablet core (i.e., the pharmaceutical composition of the present disclosure).

A third aspect of the present disclosure provides a preparation method for the pharmaceutical composition described above, which comprises:
premixing the compound (A) with another excipient other than a lubricant, and mixing the obtained premix with a lubricant to obtain a pharmaceutical composition comprising the compound (A);
optionally and further, filling the obtained pharmaceutical composition comprising the compound (A) into a capsule or compressing the obtained pharmaceutical composition comprising the compound (A) into a tablet.

In some embodiments of the present disclosure, the premixing may be performed in multiple steps. Further, the premixing further comprises a deagglomeration step, for example, premixing one or more materials with a glidant, and then performing deagglomeration one or more times.

In some embodiments of the present disclosure, the preparation method further comprises a step of adding an acidic pH regulator, wherein the acidic pH regulator is selected from oxalic acid, maleic acid, fumaric acid, glutaric acid, and the like.

A fourth aspect of the present disclosure provides use of the pharmaceutical composition described above or the solid oral formulation described above for the manufacturing of a medicament for the prevention and/or treatment of a disease associated with activity or expression of FGFR, KDR, and/or CSF-1R. Preferably, the disease is a tumor. Further preferably, the tumor is selected from the group consisting of: breast cancer, lung cancer, non-small cell lung cancer, bladder cancer, urinary tract transitional cell carcinoma, esophageal cancer, gastric cancer (including gastroesophageal junction cancer), pancreatic cancer, prostate cancer, colorectal cancer, myeloma, multiple myeloma, acute myeloid leukemia, liver cancer, melanoma, thyroid cancer, head and neck cancer, renal cell carcinoma, glioblastoma, squamous cell carcinoma, esophageal squamous cell carcinoma, squamous lung carcinoma, nasopharyngeal squamous carcinoma, testicular cancer, and cholangiocarcinoma.

A fifth aspect of the present disclosure provides the pharmaceutical composition described above or the solid oral formulation described above for use in the prevention and/or treatment of a disease associated with activity or expression of FGFR, KDR, and/or CSF-1R. Preferably, the disease is a tumor. Further preferably, the tumor is selected from the group consisting of: breast cancer, lung cancer, non-small cell lung cancer, bladder cancer, urinary tract transitional cell carcinoma, esophageal cancer, gastric cancer (including gastroesophageal junction cancer), pancreatic cancer, prostate cancer, colorectal cancer, myeloma, multiple myeloma, acute myeloid leukemia, liver cancer, melanoma, thyroid cancer, head and neck cancer, renal cell carcinoma, glioblastoma, squamous cell carcinoma, esophageal squamous cell carcinoma, squamous lung carcinoma, nasopharyngeal squamous carcinoma, testicular cancer, and cholangiocarcinoma.

A sixth aspect of the present disclosure provides a method of prevention and/or treatment of a disease associated with activity or expression of FGFR, KDR, and/or CSF-1R, which comprises administering to a subject in need thereof an effective dose of the pharmaceutical composition described above or the solid oral formulation described above. Preferably, the disease is a tumor. Further preferably, the tumor is selected from the group consisting of: breast cancer, lung cancer, non-small cell lung cancer, bladder cancer, urinary tract transitional cell carcinoma, esophageal cancer, gastric cancer (including gastroesophageal junction cancer), pancreatic cancer, prostate cancer, colorectal cancer, myeloma, multiple myeloma, acute myeloid leukemia, liver cancer, melanoma, thyroid cancer, head and neck cancer, renal cell carcinoma, glioblastoma, squamous cell carcinoma, esophageal squamous cell carcinoma, squamous lung carcinoma, nasopharyngeal squamous carcinoma, testicular cancer, and cholangiocarcinoma.

"Subject" described herein is used interchangeably with "patient", and includes all members of the animal kingdom, including but not limited to non-human mammals (e.g., mice, rats, cats, monkeys, dogs, horses, pigs, etc.) and humans, preferably humans.

"Effective dose" described herein refers to an amount calculated based on the compound of formula (A) that is sufficient to achieve the desired therapeutic effect in a "subject" or "patient" when administered in a single dose or multiple doses.

In the scope of the present application, various options of any feature can be combined with various options of other features to form a plurality of different embodiments. The present application is intended to include all possible embodiments consisting of various options of all technical features. It is to be understood that within the scope of the present disclosure, the various technical features described above of the present disclosure and the technical features specifically described hereinafter (as in the examples) may be combined with each other to constitute a new or preferred technical scheme.

The pharmaceutical composition of the present disclosure has the following beneficial effects:
(1) The pharmaceutical composition of the present disclosure comprises the compound (A) as an active ingredient. Compared to the free base compound (I), a salt formation form thereof, the compound (A), has advantages of being easy to dry, good solubility, stable quality, thermodynamic stability, and ease of druggability, and is more suitable for use as an active ingredient in a pharmaceutical composition;
(2) the pharmaceutical composition of the present disclosure has a good active ingredient dissolution;
(3) the pharmaceutical composition of the present disclosure has qualified active ingredient content uniformity;
(4) the pharmaceutical composition of the present disclosure is conducive to providing a solid oral formulation with stable properties and suitable for long-term storage; and
(5) the preparation method for the pharmaceutical composition of the present disclosure is simple and convenient for industrial large-scale production.

### DETAILED DESCRIPTION

The present disclosure will be further illustrated with reference to the following specific examples. It should be understood that these examples are merely intended to illustrate the present disclosure rather than limit the scope of the present disclosure. Experimental methods without specified conditions in the following examples are conducted under conventional conditions or conditions recommended by the manufacturer.

In the following examples, the related analysis and detection conditions are as follows:
1. X-ray powder diffractometer (XRPD)
Instrument: BRUKER D8 Advance X-ray powder diffractometer, Germany.
Conditions: Cu-Kα radiation (λ = 1.5418 Å), tube voltage: 40 kV, tube current: 40 mA, 2θ scanning range: 3-40°, scanning step size: 0.02° (2θ), scanning speed: 10 s/step, sample tray: a zero background sample tray.
Method: a sample was spread on a zero background monocrystalline silicon sample tray and was gently pressed using a medicine spoon to be flat for measurement.

2. Chloride
Detection instrument: Dionex ICS-900 ion chromatograph
Column: Dionex Ion Pac AS11-HC anion chromatographic column (specification: 4 × 250 mm)
Determination method: 10 µL of each of the control solution and the sample solution was measured precisely and injected into the ion chromatograph, the chromatogram was recorded, and the chloride ion content was calculated based on peak area according to the external standard method.

3. Dynamic vapor sorption/desorption (DVS) analysis
Instrument: DVS Intrinsic plus (SMS, UK).
Method: a sample was placed in a tared sample basket, the weight of the sample was automatically measured, and the sample was analyzed according to the parameters in the table below.

| | |
|---|---|
| Instrument information | SMS, DVS Intrinsic PLUS |
| dm/dt | 0.002%/min |
| Drying/testing temperature | 40 °C/25 °C |
| Cycle | Full cycle |
| dm/dt minimum equilibration time | 30 min |
| dm/dt maximum equilibration time | 120 min |
| Data storage rate | 5 s |
| Gas and flow rate | N₂, 200 sccm |
| Post-run flow rate | 200 sccm |
| Step size | 10% RH |
| Method | Sorption: 0, 10, 20, 30, 40, 50, 60, 70, 80, 90 |
| | Desorption: 80, 70, 60, 50, 40, 30, 20, 10, 0 |

4. Detection conditions for thermogravimetric analysis (TGA)
Instrument: Discovery TGA 55.
Conditions: temperature range: room temperature to 350 °C; heating rate: 10 °C/min; purging gas: nitrogen; flow rate in equilibration chamber: 40 mL/min; flow rate in sample chamber: 25 mL/min.
Sample: 1-5 mg.

5. Conditions for content determination
Instrument: high performance liquid chromatograph (Waters e2689 2495)
Chromatographic column: Waters Symmetry C18, 250 × 4.6 mm, 5 µm
Mobile phase: 10 mmol/L ammonium acetate solution-acetonitrile (40:60) Injection volume: 10 µL

6. Conditions for dissolution determination
Instrument: high performance liquid chromatograph (Waters e2689 2495)
Chromatographic column: Waters XBridge C18, 250 × 4.6 mm, 5 µm
Mobile phase: 10 mmol/L ammonium acetate solution-acetonitrile (60:40)
Injection volume: 20 µL
Dissolution method: paddle method
Rotation speed: 50 rpm
Dissolution medium: 1000 mL of a potassium dihydrogen phosphate-sodium hydroxide buffer at pH 6.0
Medium temperature: 37±0.5 °C

### Preparation Example 1: Preparation of Compound A

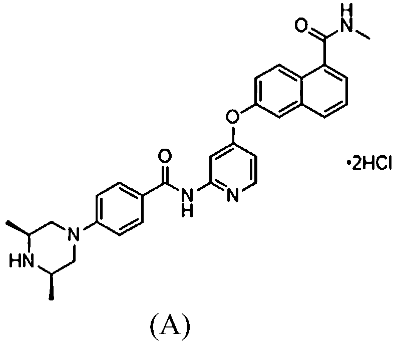

The compound (I) (0.52 g, prepared according to Preparation Example 10 of WO2017140269A1) and methanol (25 mL) were added to a reaction flask and stirred, and after the system was dispersed uniformly, a methanolic hydrochloric acid solution (1.2 mL, 2 mol/L) was added dropwise under stirring. After the dropwise addition was completed, the mixture was successively stirred for 5 h. Then, the mixture was filtered and dried under vacuum (dried under vacuum at 30 °C for 12 h until a constant weight was achieved, and then baked under vacuum at 45 °C until the solvent residue was qualified) to obtain a solid (0.53 g). Nuclear magnetic resonance detection confirmed the formation of the salt. By measuring the chloride ion content using ion chromatography, the stoichiometric ratio of the hydrochloride was calculated (see Table 1 below), and the base/acid ratio of the hydrochloride was concluded to be 1:2.

**Table 1**

| Name | Theoretical stoichiometric ratio (base/acid) | Theoretical chloride ion content (%) | Measured chloride ion content (%) |
|---|---|---|---|
| Preparation Example 1 | 1:2 | 12.2% | 11.7% |

The obtained solid sample was taken and subjected to X-ray powder diffraction, and the results demonstrated that the solid sample had good crystallinity. It was designated as crystalline form I, with its diffraction peak data shown in Table 2.

**Table 2 XRPD diffraction peak data of crystalline form I sample obtained in Preparation Example 1**

| 2θ (°) | Relative peak intensity (%) | 2θ (°) | Relative peak intensity (%) | 2θ (°) | Relative peak intensity (%) |
|---|---|---|---|---|---|
| 4.964 | 100.0 | 18.012 | 7.8 | 23.857 | 13.0 |
| 9.960 | 7.4 | 19.318 | 24.9 | 24.246 | 20.4 |
| 10.371 | 17.1 | 20.048 | 8.5 | 25.179 | 55.9 |
| 11.192 | 14.0 | 20.460 | 43.5 | 26.555 | 12.4 |
| 13.485 | 93.9 | 21.394 | 97.3 | 28.539 | 14.8 |
| 14.015 | 11.0 | 21.943 | 10.6 | 30.222 | 9.3 |
| 15.704 | 5.5 | 22.460 | 20.3 | 31.974 | 5.8 |
| 17.326 | 4.3 | 23.340 | 22.2 | 33.014 | 6.0 |

### Preparation Example 2: Preparation of Crystalline Form I of Compound (A)

The compound (I) (1.0432 g, prepared according to Preparation Example 10 of WO2017140269A1) and purified water (3 mL) were added to a reaction flask and stirred until the system was dispersed uniformly. An aqueous hydrochloric acid solution (2 N) was added dropwise, and the mixture was stirred until complete dissolution. The crystalline form I sample (0.01 g) obtained in Preparation Example 1 was added as a crystal seed, and the resulting mixture was stirred for crystallization for 20-24 h and then subjected to suction filtration. The filter cake was dried under vacuum at 30 °C for 12 h and then baked under vacuum at 45 °C until the solvent residue was qualified, thus obtaining a solid (1.112 g). The solid was detected, confirming that the crystalline form I of the compound (A) was obtained, with good crystallinity. The XRPD characterization data of the sample obtained are shown in Table 3. The sample was subjected to a TGA-DSC test, and the results demonstrated that the sample did not contain water of crystallization or solvent of crystallization.

**Table 3 XRPD diffraction peak data of crystalline form I sample obtained in Preparation Example 2**

| 2θ(°) | Relative peak intensity (%) | 2θ (°) | Relative peak intensity (%) | 2θ (°) | Relative peak intensity (%) |
|---|---|---|---|---|---|
| 4.955 | 37.7 | 19.305 | 31.6 | 25.246 | 50.5 |
| 9.982 | 5.20 | 20.075 | 8.5 | 26.572 | 14.9 |
| 10.384 | 13.7 | 20.496 | 41.1 | 28.654 | 8.4 |
| 11.212 | 10.4 | 21.397 | 87.5 | 30.056 | 6.5 |
| 13.503 | 100 | 22.015 | 10.2 | 32.071 | 6.7 |
| 14.056 | 9.9 | 22.526 | 17.7 | 33.032 | 4.8 |
| 15.733 | 7 | 23.367 | 22.8 | | |
| 17.382 | 5.6 | 23.884 | 17 | | |
| 18.029 | 4.4 | 24.298 | 17.7 | | |

.

### Preparation Example 3. Preparation of Other Salts of Compound (I)

To each of flasks containing 5 mL of a 0.02 mol/mL solution of an acid (phosphoric acid, maleic acid, tartaric acid, and fumaric acid) in tetrahydrofuran/methanol (1:1, volume ratio) was added 10 mL of a 0.01 mol/mL solution of compound (I) in tetrahydrofuran/methanol (1:1, volume ratio), and the mixtures were stirred homogeneously, followed by reaction at 40 °C for 1 h. The reaction liquids were volatilized and dried at 50 °C for 3-4 h to obtain solids. Solids were confirmed by nuclear magnetic resonance and ion chromatography to be the phosphate, the maleate, the tartrate, and the fumarate of the compound (I), respectively.

Further XRPD detection demonstrated that the crystallinity of the obtained solids was good.

Test Example 1: Solubility Experiment of Compound (I) and Salts Thereof Firstly, the solubility of the compound (I), the sample obtained in Preparation Example 2 (compound(A)), and the samples obtained in Preparation Example 3 was measured in water, separately. Then, the solubility of the compound (I) and the sample (compound (A)) obtained in Preparation Example 2 was measured in a Na₂HPO₄-citric acid buffer medium (pH 4.6), separately. The experimental results are shown in Tables 4 and 5.

**Table 4 Solubility experimental results of compound (I) and different salt forms thereof in water (25 °C, mg/mL)**

| Solvent/salt | Compound (I) | Compound (A) | Phosphate | Maleate | Tartrate | Fumarate |
|---|---|---|---|---|---|---|
| Deionized water | 0.008 | 16.2 | 3.5 | 2.0 | 1.4 | 1.3 |

**Table 5 Solubility experimental results of compound (I) and compound (A) in buffer (25 °C, mg/mL)**

| Solvent/salt | Compound (I) | Compound (A) |
|---|---|---|
| Na₂HPO₄-citric acid buffer (pH 4.6) | 0.045 | 0.1 |

The results showed that: the compound (A) obtained in Preparation Example 2 and the samples of different salts obtained in Preparation Example 3 had good solubility in water, and their solubility was significantly superior to that of the free base, which met the general requirement of oral dosage forms for the solubility of drug substances; the compound (A) in particular exhibited excellent solubility, which met the general requirement of various pharmaceutical dosage forms (e.g., solid oral dosage forms, injections, oral liquids, etc.) for the solubility of drug substances. The compound (A) also had better solubility in the buffer than the compound (I).

### Test Example 2: Water Sorption and Desorption Experiments of Different Salt Forms of Compound (I)

The samples obtained in Preparation Example 2 and Preparation Example 3 were investigated for moisture sorption and desorption under the relative humidity of 40-80% using a dynamic vapor sorption (DVS) instrument at a temperature of 25 °C to determine the hygroscopicity of various salt forms. The experimental results are shown in Table 6.

**Table 6 Hygroscopicity experimental results of different salt forms of compound (I) (DVS, 40-80%)**

| Salt form | Compound (A) | Phosphate | Maleate | Tartrate | Fumarate |
|---|---|---|---|---|---|
| Hygroscopicity (%) | 0.65 | 3.75 | 0.81 | 2.86 | 2.11 |

The results showed that: the compound (A) was slightly hygroscopic, and the hygroscopic weight gain was relatively low.

### Test Example 3: Solid Stability Experiment of Compound (A)

The sample obtained in Preparation Example 2 was investigated for physical stability and chemical stability under the conditions of 40 °C/75% RH (open) and 60 °C (sealed) for 7 days, and for physical stability under the conditions of room temperature/92.5% RH (open) for 10 days. The experimental results are shown in Table 7 below.

**Table 7 Solid stability experiment results of compound (A)**

| Parameter/condition | Initial state | 40 °C/75% RH | 60 °C | 92.5% RH |
|---|---|---|---|---|
| | 0 days | 7 days | 7 days | 10 days |
| Purity/% | 99.85 | 99.86 | 99.85 | / |
| Crystalline form | Crystalline form I | Crystalline form I | Crystalline form I | Crystalline form I |

| | | | | |
|---|---|---|---|---|
| Note: the "/" indicates undetected; the crystalline form results were obtained using the X-ray powder diffraction method. | | | | |

The results showed that: after compound (A) was left to stand under the conditions of 40 °C/75% RH and 60 °C for 7 days, the purity thereof showed no significant changes, and the crystalline form thereof was not changed; after compound (A) was left to stand under the condition of 92.5% RH for 10 days, the crystalline form thereof was also not changed. The results described above showed that the compound (A) has good chemical stability and physical stability.

### Test Example 4: Grinding Stability Experiment of Compound (A)

The sample obtained in Preparation Example 2 was mechanically ground using a mortar for 2 min and 5 min, separately, and X-ray powder diffraction detection demonstrated that no crystalline form change occurred in the crystalline form I after grinding.

Furthermore, the sample obtained in Preparation Example 2 was mechanically ground after a suitable amount of each organic solvent (methanol, ethanol, acetone, acetonitrile, or ethyl acetate) was separately added dropwise, and X-ray powder diffraction detection demonstrated that no crystalline form change occurred in the crystalline form I after grinding.

The active ingredient compound (A) used in the following examples was the crystalline form I.

The limit requirements of investigation items for tablets comprising the compound (A) prepared in the following examples are shown in Table 8:

**Table 8 Limit requirements for investigation items of tablets of examples**

| Investigation item | Limit requirement |
|---|---|
| Related substances (%) | Total impurities ≤ 1.5 |
| Dissolution (%) | No less than 75% of the labeled amount at 30 min |
| Content determination (%) | Should be 90.0%-110.0% of the labeled amount |
| Content uniformity A+2.2 S | ≤15 |

### Example 1: Preparation of Tablet (51 mg/Tablet) Comprising Compound (A)

The formula of the tablet of Example 1 is shown in Table 9 below:

**Table 9 Formula of Example 1**

| Component | Amount (mg/tablet) | Percentage (%) | Effect |
|---|---|---|---|
| Compound (A) | 2.00 | 3.9 | Active ingredient |
| Mannitol | 10.20 | 20.0 | Filler |
| Microcrystalline cellulose | 29.20 | 57.2 | Filler |
| Pregelatinized starch | 5.34 | 10.5 | Filler |
| Crospovidone | 2.00 | 3.9 | Disintegrant |
| Colloidal silica | 0.75 | 1.5 | Glidant |
| Glyceryl behenate | 1.51 | 3.0 | Lubricant |
| Total | 51 | 100.0 | -- |

### Preparation method:

1 Weighing: the raw materials and excipients were accurately weighed according to the amounts in the formula in Table 9;
2 Premixing:
   the colloidal silica and the mannitol were mixed in a bag, sieved through a 40-mesh sieve, and divided into two portions, wherein the first portion was about 6% of the total weight, and the second portion was 94%;
   the pregelatinized starch, the compound (A), the crospovidone, and the first portion of the mixture of the colloidal silica and the mannitol were added into a container of an appropriate volume and mixed, followed by the addition of about half of the microcrystalline cellulose to the container for mixing;
   the remaining microcrystalline cellulose and the second portion of the mixture of the colloidal silica and the mannitol were added to the container described above and mixed;
3 Total mixing: the glyceryl behenate was added in an equal incremental manner and mixed;
4 Tableting: the mixture obtained in step 3 was tableted according to a theoretical weight of 51 mg/tablet; the difference in tablet weight was ±7.5%; the hardness was 4-10 kg.

The prepared tablet was investigated for the dissolution (phosphate buffer at pH 6.0), assay, content uniformity, and process feasibility, and the results are shown in Table 10 below:

**Table 10 Test results of tablet of Example 1**

| Sample | Dissolution rate (30 min) mean% (n = 6) | Mean Assay % | Content uniformity A+2.2 S | Process feasibility (sticking/picking) |
|---|---|---|---|---|
| Example 1 | 89 | 99.8 | 5.9 | No |

The results showed that: the tablet comprising the compound (A) as the active ingredient was prepared by adopting the formula of this example, the tableting process was smooth, and the dissolution and content uniformity of the prepared tablet sample met the requirements; the preparation process is simple and highly feasible.

### Example 2: Preparation of Tablet (100 mg/Tablet) Comprising Compound (A)

The formula of the tablet of Example 2 is shown in Table 11 below:

**Table 11. Tablet formula of Example 2**

| Component | Amount (mg/tablet) | Percentage (%) | Effect |
|---|---|---|---|
| Compound (A) | 2.0 | 2.0 | Active ingredient |
| Mannitol | 20.0 | 20.0 | Filler |
| Microcrystalline cellulose | 61.0 | 61.0 | Filler |
| Pregelatinized starch | 10.5 | 10.5 | Filler |
| Crospovidone | 4.0 | 4.0 | Disintegrant |
| Colloidal silica | 1.5 | 1.5 | Glidant |
| Sodium stearyl fumarate | 1.0 | 1.0 | Lubricant |
| Total | 100 | 100.00 | -- |

### Preparation method:

1 Pretreatment of drug substance: the compound (A) was sieved through a 100-mesh sieve, and the undersize fraction was collected.
2 Weighing: the raw materials and excipients were accurately weighed according to the amounts in the formula in Table 11;
3 Premixing:
   premixing I: the mannitol, the compound (A), the colloidal silica, and the crospovidone were added to an appropriate container and mixed;
   deagglomeration I: the mixture obtained from premixing I and the pregelatinized starch were added to a granulator or sieved for deagglomeration;
   premixing II: the mixture obtained from deagglomeration I was added to a container and mixed;
   premixing III: the microcrystalline cellulose and the premix obtained from premixing II were added to another container of an appropriate volume and mixed;
   deagglomeration II: the mixture obtained from premixing III was passed through a granulator or sieved for deagglomeration;
   premixing IV: the mixture obtained from deagglomeration II was added to a container and mixed;
4 Total mixing:
   the sodium stearyl fumarate was mixed with about an equal amount of the premixing IV mixture, sieved through a 24-mesh sieve, and then added to the container holding the premixing IV mixture for mixing.
5 Tableting: the mixture obtained in step 4 was tableted according to a theoretical tablet weight of 0.1 g/tablet; the difference in tablet weight was ±7.5%; the hardness was 3-12 kg.

The prepared tablet was investigated for the dissolution(phosphate buffer at pH 6.0), assay, content uniformity, and process feasibility, and the results are shown in Table 12 below:

**Table 12 Test results of tablet of Example 2**

| Sample | Dissolution (30 min) mean% (n = 6) | Mean assay % | Content uniformity A+2.2 S | Process feasibility (sticking/picking) |
|---|---|---|---|---|
| Example 2 | 94 | 100.1 | 4.5 | No |

The results showed that: the tablet prepared according to the formula of this example comprising the compound (A) as the active ingredient exhibited a good dissolution and qualified content uniformity; the preparation process was highly feasible.

### Examples 3-7: Preparation of Tablets Comprising Compound (A) (Investigation of Amount of Filler)

(1) Investigation of weight ratio of mannitol to microcrystalline cellulose Tablets of Examples 3-5 were prepared by referring to the formula and preparation method of Example 2, with only the weight ratio of the mannitol to the microcrystalline cellulose changed. The amounts and ratios of the mannitol and the microcrystalline cellulose in Examples 3-5 are shown in Table 13 below.

**Table 13 Amounts and ratios of mannitol to microcrystalline cellulose in Examples 3-5**

| Example | Amount of mannitol (mg/tablet) | Microcrystalline cellulose (mg/tablet) | Ratio (mannitol: microcrystalline cellulose) |
|---|---|---|---|
| 3 | 27 | 54 | 1:2 |
| 4 | 40.5 | 40.5 | 1:1 |
| 5 | 54 | 27 | 2:1 |

The tablets prepared in Examples 3-5 were investigated for the dissolution (phosphate buffer at pH 6.0), assay, content uniformity, and process feasibility, and the results are shown in Table 14 below:

**Table 14 Test results of tablets of Examples 3-5**

| Sample | Dissolution (30 min) mean% (n = 6) | Mean assay % | Content uniformity A+2.2 S | Process feasibility (sticking/picking) |
|---|---|---|---|---|
| Example 3 | 96 | 102.1 | 8.6 | No |
| Example 4 | 95 | 99.8 | 3.5 | No |
| Example 5 | 96 | 100.8 | 17.7 | No |

The results showed that: the tablets prepared by using the mannitol and the microcrystalline cellulose in different ratios as fillers all exhibited good active ingredient dissolution, and the process was highly feasible; but the active ingredient content uniformity of the tablets prepared when the ratio of the mannitol to the microcrystalline cellulose was 2:1 was unqualified.
(2) Investigation of amount of pregelatinized starch
Tablets of Examples 6-7 were prepared by referring to the formula and preparation method of Example 2, with the amount of the pregelatinized starch changed and the amount of the microcrystalline cellulose adjusted to ensure that the amount of the filler and the total weight of the tablet remained unchanged. The amounts of the pregelatinized starch in Examples 6-7 are shown in Table 15 below.

**Table 15 Amounts of pregelatinized starch in Examples 6-7**

| Sample | Example 6 | Example 7 |
|---|---|---|
| Amount of pregelatinized starch (mg/tablet) | 5 | 15 |

The tablets prepared in Examples 6-7 were investigated for the dissolution (phosphate buffer at pH 6.0), assay, content uniformity, and process feasibility, and the results are shown in Table 16 below:

**Table 16 Test results of tablets of Examples 6-7**

| Sample | Dissolution (30 min) mean% (n = 6) | Mean assay % | Content uniformity A+2.2 S | Process feasibility (sticking) |
|---|---|---|---|---|
| Example 6 | 95 | 100.9 | 5.3 | No |
| Example 7 | 99 | 100.0 | 3.6 | No |

The results showed that: at the screened amount of the pregelatinized starch, the prepared tablets all exhibited good active ingredient dissolution and good active ingredient content uniformity, and the process was highly feasible.

### Examples 8-10: Preparation of Tablets Comprising Compound (A) (Investigation of Amount of Disintegrant)

Tablets of Examples 8-10 were prepared by referring to the formula and preparation method of Example 2, with only the amount of the disintegrant changed (the total tablet weight was changed accordingly). The amounts of the disintegrant in Examples 8-10 are shown in Table 17 below.

**Table 17 Amounts of disintegrant in Examples 8-10**

| Sample | Example 8 | Example 9 | Example 10 |
|---|---|---|---|
| Amount of crospovidone (mg/tablet) | 0 | 2 | 6 |

The tablets prepared in Examples 8-10 were tested for the dissolution (phosphate buffer at pH 6.0), and the results are shown in Table 18 below:

**Table 18 Dissolution test results of tablets of Examples 8-10**

| Sample | Example 8 | Example 9 | Example 10 |
|---|---|---|---|
| Dissolution (30 min) mean% (n = 6) | 85 | 91 | 99 |

The results showed that: the tablets of Example 8 containing no disintegrant also exhibited an acceptable active ingredient dissolution; the addition of the disintegrant further improved the active ingredient dissolution, as shown in Examples 9-10.

### Examples 11-14: Preparation of Tablets Comprising Compound (A) (Investigation of Amount of Glidant)

Tablets of Examples 11-12 were prepared by referring to the formula and preparation method of Example 2, with only the amount of the glidant changed (the total tablet weight was changed accordingly).

Tablets of Examples 13-14 were prepared by referring to the formula and preparation method of Example 1, with the ratio of the mannitol to the microcrystalline cellulose adjusted to 1:1 and the amount of the glidant changed. The amounts of the glidant in Examples 11-14 are shown in Table 19 below.

**Table 19 Amounts of glidant in Examples 11-14**

| Sample | Example 11 | Example 12 | Example 13 | Example 14 |
|---|---|---|---|---|
| Amount of Colloidal silica (mg/tablet) | 0 | 3.0 | 1.47 | 2.88 |

The tablets prepared in Examples 11-14 were tested (n = 6) for the assay and content uniformity, and the results are shown in Table 20 below:

**Table 20 Test results of tablets of Examples 11-14**

| Sample | Mean assay % | Content uniformity A+2.2 S |
|---|---|---|
| Example 11 | 103.1 | 6.4 |
| Example 12 | 101.2 | 6.9 |
| Example 13 | 102.7 | 9.5 |
| Example 14 | 103.1 | 6.6 |

The results showed that: at the tested amount of the glidant, the prepared tablets all exhibited qualified content uniformity.

### Examples 15-16: Preparation of Tablets Comprising Compound (A) (Investigation of Amount of Lubricant)

Tablets of Examples 15-16 were prepared by referring to the formula and preparation method of Example 2, with only the amount of the lubricant changed (the total tablet weight was changed accordingly). The amounts of the lubricant in Examples 15-16 are shown in Table 21 below.

**Table 21 Amounts of lubricant in Examples 15-16**

| Sample | Example 15 | Example 16 |
|---|---|---|
| Amount of sodium stearyl fumarate (mg/tablet) | 0.8 | 1.5 |

The tablets prepared in Examples 15-16 were tested for the dissolution (phosphate buffer at pH 6.0) and process feasibility, and the results are shown in Table 22 below:

**Table 22: Test results for tablets of Examples 15-16**

| Sample | Dissolution (30 min) mean% (n = 6) | Process feasibility (sticking/picking) |
|---|---|---|
| Example 15 | 93 | No |
| Example 16 | 88 | No |

The results showed that: at the tested amount of the lubricant, the prepared tablets all exhibited good dissolution, and the preparation process was highly feasible.

### Examples 17-19: Preparation of Film-coated Tablets Comprising Compound (A) (Investigation of Coating Weight Gain)

Tablets comprising the compound (A) were prepared by referring to the formula and preparation method of Example 2, and coated with a film coating premix (gastrosoluble)-Opadry^{®} 321A610052 in which the solid content of the coating solution was 30%, and the weight gain was controlled in the range of 2.0%-4.0% of the tablet core weight, to prepare film-coated tablets of Examples 17-19. The coating weight gains % in Examples 17-19 are shown in Table 23 below.

**Table 23 Coating weight gains in Examples 17-19**

| Sample | Example 17 | Example 18 | Example 19 |
|---|---|---|---|
| Coating weight gain % | 2 | 3 | 4 |

The film-coated tablets prepared in Examples 17-19 were tested for the dissolution (phosphate buffer at pH 6.0) and observed for appearance, and the results are shown in Table 24 below:

**Table 24 Dissolution results and appearance of film-coated tablets of Examples 17-19**

| Sample | Dissolution (30 min) mean% (n = 6) | Appearance |
|---|---|---|
| Example 17 | 91 | Complete coating film with uniform color |
| Example 18 | 91 | Complete coating film with uniform color |
| Example 19 | 91 | Complete coating film with uniform color |

The results showed that: the film-coated tablets obtained after coating still exhibited good active ingredient dissolution and good appearance.

### Examples 20-21: Other Preparation Examples of Tablets Comprising Compound (A)

Tablets of Examples 20-21 were prepared by referring to the preparation method of Example 1, according to the formulas in Table 25 below.

**Table 25 Formulas of tablets of Examples 20-21**

| Component | Component weight percentage (%) | |
|---|---|---|
| | Example 20 | Example 21 |
| Compound (A) | 3.9 | 3.9 |
| Mannitol | 38.1 | 25 |
| Microcrystalline cellulose | 39.1 | 42.2 |
| Pregelatinized starch | 10.5 | 21 |
| Crospovidone | 3.9 | 3.9 |
| Colloidal silica | 1.5 | 1.5 |
| Glyceryl behenate | 3.0 | 2.5 |

The tablets prepared in Examples 20-21 were investigated for the dissolution (phosphate buffer at pH 6.0), assay, content uniformity, and process feasibility, and the results are shown in Table 26 below:

**Table 26 Test results of tablets of Examples 20-21**

| Sample | Dissolution (30 min) mean% (n = 6) | Mean assay % | Content uniformity A+2.2 S | Process feasibility (sticking) |
|---|---|---|---|---|
| Example 20 | 93 | 100.5 | 10.2 | No |
| Example 21 | 99 | 98.9 | 6.1 | No |

### Examples 22-24: Preparation of Tablets Comprising Compound (A) (Investigation of Different Lubricants)

Tablets of Examples 22-24 were prepared by referring to the preparation method of Example 2, according to the formulas shown in Table 27 below.

**Table 27 Formulas of tablets of Examples 22-24**

| Component | Component weight percentage (%) | | |
|---|---|---|---|
| | Example 22 | Example 23 | Example 24 |
| Compound (A) | 2.0 | 2.0 | 2.0 |
| Mannitol | 20.0 | 20.0 | 20.0 |
| Microcrystalline cellulose | 59.1 | 59.1 | 59.1 |
| Pregelatinized starch | 10.5 | 10.5 | 10.5 |
| Crospovidone | 3.9 | 3.9 | 3.9 |
| Colloidal silica | 1.5 | 1.5 | 1.5 |
| Stearic acid | 3.0 | - | - |
| Magnesium stearate | - | 3.0 | - |
| Sodium stearyl fumarate | - | - | 3.0 |

The stability of the tablets prepared in Examples 2 and 22-24 was investigated at 60 °C for 1 month, and the results are shown in Table 28 below:

**Table 28 Stability test results for tablets of Examples 2 and 22-24**

| Sample | Total impurities % on day 0 | Total impurities % at 60 °C for 1 month |
|---|---|---|
| Example 2 | 0.12 | 0.22 |
| Example 22 | 0.07 | 0.76 |
| Example 23 | 0.07 | 0.25 |
| Example 24 | 0.13 | 0.28 |

The results showed that: under the condition of standing at 60 °C for 1 month, the total impurities of the tablets prepared using the stearic acid as the lubricant slightly increased, and the total impurities of the tablets prepared using the magnesium stearate and the sodium stearyl fumarate as the lubricants were not significantly different from those on day 0. Tablets prepared using the three lubricants after being left to stand at 60 °C for 1 month all met the specifications for total impurities.

### Examples 25-28: Preparation of Tablets Comprising Compound (A) (Investigation of Different Fillers)

Tablets of Examples 25-28 were prepared by referring to the preparation method of Example 2, according to the formulas in Table 29 below.

**Table 29 Formulas of tablets of Examples 25-28**

| | Component weight percentage (%) | | | |
|---|---|---|---|---|
| Component | Example 25 | Example 26 | Example 27 | Example 28 |
| Compound (A) | 2.0 | 2.0 | 2.0 | 2.0 |
| Mannitol | - | 20.0 | 20.0 | - |
| Lactose | - | - | - | 40.5 |
| Microcrystalline cellulose | 91.5 | 71.5 | - | 40.5 |
| Silicified microcrystalline cellulose | - | - | 62.5 | - |
| Pregelatinized starch | - | - | 10.5 | 10.5 |
| Crospovidone | 4.0 | 4.0 | 4.0 | 4.0 |
| Colloidal silica | 1.5 | 1.5 | - | 1.5 |
| Sodium stearyl fumarate | 1.0 | 1.0 | 1.0 | 1.0 |

The tablets prepared in Examples 25-28 were investigated for the dissolution (phosphate buffer at pH 6.0), assay, content uniformity, and process feasibility, and the results are shown in Table 30 below:

**Table 30 Test results of tablets of Examples 25-28**

| Sample | Dissolution (30 min) mean% (n = 3) | Mean assay % | Content uniformity A+2.2 S | Process feasibility (sticking/picking) |
|---|---|---|---|---|
| Example 25 | 79 | 98.6 | 7.5 | No |
| Example 26 | 86 | 98.4 | 6.0 | No |
| Example 27 | 88 | 101.8 | 4.9 | No |
| Example 28 | 91 | 97.7 | 7.9 | No |

The results showed that: the tablets prepared using the microcrystalline cellulose as the sole filler (Example 25), or combinations of the microcrystalline cellulose with other fillers, such as the combination of the microcrystalline cellulose and the mannitol (Example 26), or the combination of the microcrystalline cellulose (or the silicified microcrystalline cellulose) with the lactose and the pregelatinized starch (Examples 27-28), all exhibited good active ingredient dissolution and qualified content uniformity.

### Examples 29-30: Preparation of Tablets Comprising Compound (A) (Investigation of Different Disintegrants)

Tablets of Examples 29-30 were prepared by referring to the preparation method of Example 2, except for replacing the disintegrant crospovidone with the disintegrants in Table 31 below.

**Table 31 Disintegrants used in Examples 29-30**

| Component | Component weight percentage (%) | |
|---|---|---|
| | Example 29 | Example 30 |
| Disintegrant | Low-substituted hydroxypropylcellulose | Sodium carboxymethyl starch |

The tablets prepared in Examples 29-30 were investigated for the dissolution (phosphate buffer at pH 6.0), and the results are shown in Table 32 below:

**Table 32 Dissolution test results of tablets of Examples 29-30**

| Sample | Dissolution (30 min) mean% (n = 3) |
|---|---|
| Example 29 | 89 |
| Example 30 | 88 |

The results showed that: the tablets with good active ingredient dissolution can also be prepared by using the low-substituted hydroxypropylcellulose and the sodium carboxymethyl starch as disintegrants.

### Example 31: Preparation of Tablet Comprising Compound (A) (Investigation of Different Glidants)

The tablet of Example 31 was prepared by referring to the formula and preparation method of Example 2, except for replacing the colloidal silica with the silica. The prepared tablet was investigated for the dissolution (phosphate buffer at pH 6.0), assay, content uniformity, and process feasibility, and the results are shown in Table 33 below:

**Table 33 Test results of tablet of Example 31**

| Sample | Dissolution (30 min) mean% (n = 3) | Mean assay % | Content uniformity A+2.2 S | Process feasibility (sticking/picking) |
|---|---|---|---|---|
| Example 31 | 83 | 96.4 | 6.3 | No |

The results showed that the tablet prepared using the silica as the glidant also exhibited a good active ingredient dissolution and good active ingredient content uniformity, and the process was highly feasible.

### Example 32: Preparation of Coated Tablet Comprising Compound (A)

A tablet comprising the compound (A) was prepared by referring to the formula and preparation method of Example 1, and coated with a film coating premix (gastrosoluble)-Opadry^{®} 03B28796 in which the solid content of the coating solution was 10%, and the weight gain was 3.7%, to prepare a film-coated tablet comprising the compound (A).

### Example 33: Preparation of Coated Tablet Comprising Compound (A)

A tablet comprising the compound (A) was prepared by referring to the formula and preparation method of Example 2, and coated with a film coating premix (gastrosoluble)-Opadry^{®} 321A610052 in which the solid content of the coating solution was 30%, and the weight gain was 2.6%, to prepare a film-coated tablet comprising the compound (A).

The prepared tablet may be double-sided scored to meet the flexibility needed for the product dose. The obtained tablet was easy to split, and the quality of the half-tablets after splitting met the specifications, see Table 34 below:

**Table 34 Test results of split half-tablets**

| Test item | | Manual splitting | Mechanical splitting |
|---|---|---|---|
| Weight difference % (n = 30) | Mean of 30 half-tablets | 52 mg | 53 mg |
| | Acceptable range of tablet weight (85%-115%) | 44-60 mg | 45-61 mg |
| | Measured single value range | 48-55 mg | 50-56 mg |
| | Conformity to specifications | Yes | Yes |
| Content uniformity A+2.2 S | | 7.6 | 8.6 |
| Dissolution (n = 12) | | 87% | 87% |

### Example 34: Preparation of Capsule Comprising Compound (A)

A pharmaceutical composition comprising the compound (A) was obtained by referring to the formula and steps 1-4 of the preparation method of Example 2, and then filled into a capsule to prepare a capsule comprising the compound (A).

### Test Example 5: Long-Term Stability Experiment

(1) The tablet prepared in Example 32 was packaged in a low-density polyethylene bottle (containing a desiccant) and left to stand at 25 °C±2°C/60%±5% RH to investigate the stability, and the experiment results are shown in Table 35 below:

**Table 35. Stability test results of tablet of Example 32 at 25 °C±2 °C/60%±5% RH**

| Investigation item | Limit requirement | Time (months) | | | | | | |
|---|---|---|---|---|---|---|---|---|
| | | 0 | 3 | 6 | 9 | 12 | 18 | 24 |
| Related substances (%) | Total impurities ≤ 1.5% | 0.20 | 0.13 | 0.20 | 0.21 | 0.19 | 0.19 | 0.18 |
| Dissolution (%) | No less than 75% of the labeled amount | 88 | 90 | 86 | 85 | 85 | 85 | 85 |
| Assay (%) | 90.0%~110.0% | 99.4 | 99.1 | 99.7 | 99.8 | 99.1 | 99.7 | 98.5 |

(2) The tablet prepared in Example 33 was packaged in an aluminum-aluminum blister pack and left to stand at 30 °C±2 °C/65%±5% RH to investigate the stability, and the experiment results are shown in Table 36 below:

**Table 36 Stability test results of tablet of Example 33 at 30 °C±2 °C/65%±5% RH**

| Investigation item | Limit requirement | Time (months) | | | | | |
|---|---|---|---|---|---|---|---|
| | | 0 | 3 | 6 | 9 | 12 | 18 |
| Related substances (%) | Total impurities ≤ 1.5 | 0.16 | 0.18 | 0.14 | 0.14 | 0.19 | 0.29 |
| Dissolution (%) | No less than 75% of the labeled amount at 30 min | 87 | 89 | 90 | 88 | 88 | 87 |
| Content determination (%) | Should be 90.0%-110.0% of the labeled amount | 99.7 | 101.4 | 102.5 | 99.0 | 100.4 | 101.7 |

The experimental results described above showed that the formulation of the present disclosure had no significant change in the related substances, dissolution, and content after being left to stand at 25 °C±2 °C/60%±5% RH for 24 months or at 30 °C±2 °C/65%±5% RH for 18 months, indicating that the formulation has stable quality and is suitable for long-term storage.

The foregoing are merely preferred embodiments of the present disclosure. It should be noted that several improvements and embellishments can be made by those of ordinary skill in the art without departing from the principles of the present disclosure, and should also be construed as within the protection scope of the present disclosure.

## Claims

1. A pharmaceutical composition, comprising the following components in percentage by weight of the pharmaceutical composition:
0.1%-10% of a compound (A) having a structure of formula (A) below 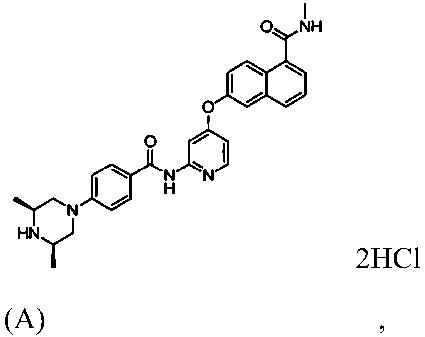
70%-95% of a filler,
0%-15% of a disintegrant,
0%-3% of a glidant, and
0.5%-5% of a lubricant.

2. The pharmaceutical composition according to claim 1, wherein the compound (A) accounts for 1%-10%, preferably 2%-8%, more preferably 2%-7%, further preferably 2%-6%, even further preferably 2%-5%, and even further preferably 2%-4% in percentage by weight of the pharmaceutical composition.

3. The pharmaceutical composition according to any one of claims 1-2, wherein the filler accounts for 75%-95%, even further preferably 80%-95%, even further preferably 85%-95%, and even further preferably 85%-92% in percentage by weight of the pharmaceutical composition.

4. The pharmaceutical composition according to any one of claims 1-3, wherein the disintegrant accounts for 1%-6%, preferably 2%-6%, more preferably 2%-5%, more preferably 2%-4%, further preferably 3%-5%, even further preferably 3.5%-4.5%, and even further preferably 3%-4% in percentage by weight of the pharmaceutical composition.

5. The pharmaceutical composition according to any one of claims 1-4, wherein the glidant accounts for 0.5-3.0%, preferably 0.5%-2.5%, more preferably 0.5%-2%, and further preferably 1-2% in percentage by weight of the pharmaceutical composition.

6. The pharmaceutical composition according to any one of claims 1-5, wherein the lubricant accounts for 0.5%-4%, preferably 1%-4%, more preferably 1%-3%, and further preferably 0.8%-3% in percentage by weight of the pharmaceutical composition.

7. The pharmaceutical composition according to any one of claims 1-6, wherein the filler is selected from one or more of microcrystalline cellulose, silicified microcrystalline cellulose, xylitol, mannitol, sorbitol, starch, pregelatinized starch, dextrin, lactose, sucrose, glucose, fructose, maltose, calcium carbonate, calcium sulfate, and dicalcium phosphate; preferably, the filler is one or more of microcrystalline cellulose, silicified microcrystalline cellulose, mannitol, lactose, pregelatinized starch, and dextrin; more preferably, the filler is one or more of microcrystalline cellulose, silicified microcrystalline cellulose, mannitol, lactose, and pregelatinized starch; further preferably, the filler is one of microcrystalline cellulose or silicified microcrystalline cellulose, a combination of one of microcrystalline cellulose or silicified microcrystalline cellulose and one of mannitol or lactose, or a combination of one of microcrystalline cellulose or silicified microcrystalline cellulose, one of mannitol or lactose, and pregelatinized starch; even further preferably, the filler is a combination of microcrystalline cellulose, mannitol, and pregelatinized starch.

8. The pharmaceutical composition according to claim 7, wherein when the filler is selected from a combination of one of microcrystalline cellulose or silicified microcrystalline cellulose and one of mannitol or lactose, the mannitol or lactose has a weight percentage of 15-50%, preferably 20-45%, more preferably 20%-40%, and further preferably 20%-30%; the microcrystalline cellulose or silicified microcrystalline cellulose has a weight percentage of 40-75%, preferably 50-75%, more preferably 60-75%, and further preferably 65-75%; the mannitol or lactose and the microcrystalline cellulose or silicified microcrystalline cellulose are in a weight ratio of 1:5 to 1:1, preferably 1:4 to 1:1, more preferably 1:4 to 1:1.5, further preferably 1:3.6 to 1:1.5, even further preferably 1:3.6 to 1:2.8, and even further preferably 1:3.6 to 1:3.

9. The pharmaceutical composition according to claim 7, wherein when the filler is selected from a combination of one of microcrystalline cellulose or silicified microcrystalline cellulose, one of mannitol or lactose, and pregelatinized starch, the mannitol or lactose has a weight percentage of 15-45%, preferably 20-45%, more preferably 20%-41%, and further preferably 20%-30%; the microcrystalline cellulose or silicified microcrystalline cellulose has a weight percentage of 35-70%, preferably 40%-65%; the mannitol or lactose and the microcrystalline cellulose or silicified microcrystalline cellulose are in a weight ratio of 1:5 to 1:1, preferably 1:4 to 1:1, more preferably 1:4 to 1:1.5, further preferably 1:3.6 to 1:1.5, even further preferably 1:3.6 to 1:2.8, even further preferably 1:3.3 to 1:2.8, and even further preferably 1:3.05; the pregelatinized starch has a weight percentage of 5-25%, preferably 5-15%, more preferably 8-12%, further preferably 10-11%, and even further preferably 10.5%.

10. The pharmaceutical composition according to any one of claims 1-9, wherein the disintegrant is selected from: sodium carboxymethyl starch, sodium carboxymethylcellulose, croscarmellose sodium, dry starch, low-substituted hydroxypropylcellulose, polyvinylpyrrolidone, crospovidone, effervescent disintegrant, polacrilin potassium, and sodium alginate; preferably, the disintegrant is sodium carboxymethyl starch, sodium carboxymethylcellulose, croscarmellose sodium, low-substituted hydroxypropylcellulose, polyvinylpyrrolidone, or crospovidone; more preferably, the disintegrant is sodium carboxymethyl starch, low-substituted hydroxypropylcellulose, or crospovidone; further preferably, the disintegrant is crospovidone.

11. The pharmaceutical composition according to any one of claims 1-10, wherein the glidant is selected from: talc, silica, and colloidal silica; preferably, the glidant is silica or colloidal silica; more preferably, the glidant is colloidal silica.

12. The pharmaceutical composition according to any one of claims 1-11, wherein the lubricant is selected from: calcium stearate, magnesium stearate, zinc stearate, talc, sodium stearyl fumarate, stearic acid, glyceryl behenate, palmitic acid, glyceryl palmitostearate, magnesium lauryl sulfate, hydrogenated vegetable oil, sodium dodecyl sulfate, magnesium dodecyl sulfate, polyethylene glycol, colloidal silicon dioxide, and aluminum hydroxide; preferably, the lubricant is glyceryl behenate, magnesium stearate, or sodium stearyl fumarate; more preferably, the lubricant is glyceryl behenate or sodium stearyl fumarate.

13. The pharmaceutical composition according to any one of claims 1-12, wherein the compound (A) is in a crystalline form.

14. The pharmaceutical composition according to claim 13, wherein the crystalline form of the compound (A) is a crystalline form I, wherein the crystalline form I has characteristic peaks at 2θ of 5.0±0.2°, 13.5±0.2°, 19.3±0.2°, 20.5±0.2°, 21.4±0.2°, and 25.2±0.2° in an X-ray powder diffraction pattern obtained using Cu-Kα radiation, or
the crystalline form I has characteristic peaks at 2θ of 5.0±0.2°, 10.4±0.2°, 13.5±0.2°, 19.3±0.2°, 20.5±0.2°, 21.4±0.2°, 23.4±0.2°, and 25.2±0.2° in an X-ray powder diffraction pattern obtained using Cu-Kα radiation, or
the crystalline form I has characteristic peaks at 2θ of 5.0±0.2°, 10.4±0.2°, 13.5±0.2°, 19.3±0.2°, 20.5±0.2°, 21.4±0.2°, 22.5±0.2°, 23.4±0.2°, 23.9±0.2°, 24.3±0.2°, and 25.2±0.2° in an X-ray powder diffraction pattern obtained using Cu-Kα radiation, or
the crystalline form I has characteristic peaks at 2θ of 5.0±0.2°, 10.4±0.2°, 11.2±0.2°, 13.5±0.2°, 19.3±0.2°, 20.5±0.2°, 21.4±0.2°, 22.0±0.2°, 22.5±0.2°, 23.4±0.2°, 23.9±0.2°, 24.3±0.2°, 25.2±0.2°, and 26.6±0.2° in an X-ray powder diffraction pattern obtained using Cu-Kα radiation, or
the crystalline form I has characteristic peaks at 2θ of 5.0±0.2°, 10.0±0.2°, 10.4±0.2°, 11.2±0.2°, 13.5±0.2°, 14.1±0.2°, 15.7±0.2°, 17.4±0.2°, 18.0±0.2°, 19.3±0.2°, 20.1±0.2°, 20.5±0.2°, 21.4±0.2°, 22.0±0.2°, 22.5±0.2°, 23.4±0.2°, 23.9±0.2°, 24.3±0.2°, 25.2±0.2°, 26.6±0.2°, 28.6±0.2°, and 30.1±0.2° in an X-ray powder diffraction pattern obtained using Cu-Kα radiation, or
the crystalline form I has characteristic peaks at 2θ of 5.0±0.2°, 10.0±0.2°, 10.4±0.2°, 11.2±0.2°, 13.5±0.2°, 14.1±0.2°, 15.7±0.2°, 17.4±0.2°, 18.0±0.2°, 19.3±0.2°, 20.1±0.2°, 20.5±0.2°, 21.4±0.2°, 22.0±0.2°, 22.5±0.2°, 23.4±0.2°, 23.9±0.2°, 24.3±0.2°, 25.2±0.2°, 26.6±0.2°, 28.6±0.2°, 30.1±0.2°, 32.0±0.2°, and 33.0±0.2° in an X-ray powder diffraction pattern obtained using Cu-Kα radiation.

15. A solid oral formulation prepared from the pharmaceutical composition according to any one of claims 1-14, wherein the solid oral formulation is a capsule or a tablet, preferably a tablet, and more preferably a film-coated tablet.

16. The solid oral formulation according to claim 15, wherein the solid oral formulation is a film-coated tablet, wherein a film coating weight gain is 1%-5%, preferably 2%-4%, e.g., 2%, 2.6%, 3%, 3.7%, or 4%, of a tablet core.

17. A preparation method for the pharmaceutical composition according to claim 1, comprising:
premixing the compound (A) with another excipient other than a lubricant, and mixing the obtained premix with a lubricant to obtain a pharmaceutical composition comprising the compound (A); optionally and further, filling the obtained pharmaceutical composition comprising the compound (A) into a capsule or compressing the obtained pharmaceutical composition comprising the compound (A) into a tablet.

18. Use of the pharmaceutical composition according to any one of claims 1-14 or the solid oral formulation according to claim 15 or 16 for the manufacturing of a medicament for the prevention and/or treatment of a disease associated with activity or expression of FGFR, KDR, and/or CSF-1R.

19. The use according to claim 18, wherein the disease is a tumor; preferably, the tumor is selected from the group consisting of: breast cancer, lung cancer, non-small cell lung cancer, bladder cancer, urinary tract transitional cell carcinoma, esophageal cancer, gastric cancer (including gastroesophageal junction cancer), pancreatic cancer, prostate cancer, colorectal cancer, myeloma, multiple myeloma, acute myeloid leukemia, liver cancer, melanoma, thyroid cancer, head and neck cancer, renal cell carcinoma, glioblastoma, squamous cell carcinoma, esophageal squamous cell carcinoma, squamous lung carcinoma, nasopharyngeal squamous carcinoma, testicular cancer, and cholangiocarcinoma.
